# EUROPEAN PATENT SPECIFICATION

(11) **EP 1 529 534 B1**
(45) Date of publication and mention of the grant of the patent: **11.07.2007**
(21) Application number: 05002085.8
(22) Date of filing: 07.11.1997
(51) Int. Cl.: A61K 38/26, A61P 3/04

(54) **Use of GLP-1 peptides**
Verwendung von GLP-1 Peptiden
Utilisation de peptide GLP-1

(30) Priority: 12.11.1996 DK 127096
(43) Date of publication of application: 11.05.2005
(62) Divisional of application: 97911155.6
(73) Proprietor: NOVO NORDISK A/S, 2880 Bagsvaerd (DK)
(72) Inventor: Knudsen, Liselotte Bjerre, 4400 Kalundborg (DK); Thim, Lars, 2820 Gentofte (DK); Judge, Martin Edward, 1300 Copenhagen K (DK); Holst, Jens Juul, 2900 Hellerup (DK); Astrup, Arne Vernon, 2930 Klampenborg (DK); Wulff, Brigitte Schellerup, 2830 Virum (DK)
(74) Representative: Stahr, Pia

(56) References cited:
- WO-A-97/31943
- WO-A-98/19698
- US-A- 5 118 666
- TURTON M D ET AL: "A ROLE FOR GLUCAGON-LIKE PEPTIDE-1 IN THE CENTRAL REGULATION OF FEEDING" 4 January 1996 (1996-01-04), NATURE, MACMILLAN JOURNALS LTD. LONDON, GB, PAGE(S) 69-72 , XP002058608 ISSN: 0028-0836 * the whole document *
- NAVARRO M ET AL: "COLOCALIZATION OF GLUCAGON-LIKE PEPTIDE-1 (GLP-1) RECEPTORS, GLUCOSE TRANSPORTER GLUT-2, AND GLUCOKINASE MRNAS IN RAT HYPOTHALAMIC CELLS: EVIDENCE FOR A ROLE OF GLP-1 RECEPTOR AGONISTS AS AN INHIBITORY SIGNAL FOR FOOD AND WATER INTAKE" JOURNAL OF NEUROCHEMISTRY, NEW YORK, NY, US, vol. 67, 1996, pages 1982-1991, XP002918977 ISSN: 0022-3042
- GUTNIZK M K ET AL: "POTENTIAL THERAPEUTIC LEVELS OF GLUCAGON-LIKE PEPTIDE I ACHIEVED INHUMANS BY A BUCCAL TABLET" 1 August 1996 (1996-08-01), DIABETES CARE, AMERICAN DIABETES ASSOCIATION, ALEXANDRIA, VA, US, PAGE(S) 843-848 , XP000614162 ISSN: 0149-5992 * the whole document *

## Description

### FIELD OF THE INVENTION

The present invention relates to the use of proglucagon 72-117 and fragments and analogues thereof in the in the suppression of appetite or induction of satiety, to compositions comprising such compounds and to a method of suppressing appetite or inducing satiety in individuals in need of such a treatment.

### BACKGROUND OF THE INVENTION

The amino acid sequence of proglucagon 72-117 is given *i*.*a*. by Bell, G. I. et al. (Nature 304 368-371 (1983)). The proglucagon fragment 72-108 is commonly referred to as GLP-1(1-37) or just GLP-1. In analogy with this, the proglucagon fragment 72-117 is in the present text also referred to as GLP-1(1-45). Proglucagon originates from preproglucagon which is synthesized *i*.*a*. in the L-cells in the distal illeum, in the pancreas and in the brain. Processing of preproglucagon to give GLP-1 occurs mainly in the L-cells. A simple system is used to describe fragments and analogues of the GLP-1 related peptide. Thus, for example, Gly⁸-GLP-1(7-37) designates a fragment of GLP-1 formally derived from GLP-1 by deleting the amino acid residues Nos. 1 to 6 and substituting the naturally occurring amino acid residue in position 8 (Ala) by Gly. In the present text, the designation "an analogue" is used to designate a peptide wherein one or more amino acid residues of the parent peptide have been substituted by another amino acid residue and/or wherein one or more amino acid residues of parent peptide have been deleted and/or wherein one or more amino acid residues have been added to the parent peptide. In the present text, the designation "amino acid residue " designates the residue of an amino acid which can be coded for by the genetic code, *i.e.* a triplet ("codon") of nucleotides. In the present text, the peptides to which the invention relates are referred to collectively as "GLP-1 peptides".

Turton, M. D. et al. (Nature 379 69-72 (1996)) have investigated the effect of GLP-1 on food intake in rats. They found a profound effect of GLP-1 on food intake when the compound was administered intracerebrovetricularly but claimed that after peripheral administration GLP-1 was inactive. Tang-Christensen, M. et al. (Am. J. Physiol. 271 (40R) 848-856 (1996)) too, found that GLP-1 had a profound effect on food intake in rats when the compound was administered intracerebrovetricularly. They claimed that peripherally administered GLP-1 had no significant effect. Also, they claimed that GLP-1(1-36)amide had no effect on food intake. Surprisingly, it has now turned out that peripherally administered GLP-1 has an effect on food intake, satiety and appetite in humans and on food intake in mice. Based on these observations it is now possible to provide a medicament and a method for the prophylaxis or treatment of diseases or disorders associated with impaired appetite regulation.

### SUMMARY OF THE INVENTION

In its broadest aspect, the present invention relates to the use of GLP-1(1-45) or a fragment or an analogue thereof or the C-terminal amide of GLP-1(1-45) or a fragment or an analogue thereof in the preparation of a medicament for peripheral administration for use in suppression of appetite or induction of satiety, to a pharmaceutical composition for peripheral administration of such a compound and to a method of treating or preventing disorders associated with impaired appetite regulation or feeling of satiety.

In a preferred embodiment, the present invention relates to the use of GLP-1(1-39) or an analogue thereof or the C-terminal amide of any of these for the preparation of a medicament for peripheral administration for use in the suppression of appetite or induction of satiety.

In a further preferred embodiment, the present invention relates to the use of GLP-1(1-38) or an analogue thereof or the C-terminal amide of any of these for the preparation of a medicament for peripheral administration for use in the suppression of appetite or induction of satiety.

In a further preferred embodiment, the present invention relates to the use of GLP-1(1-37) or an analogue thereof or the C-terminal amide of any of these for the preparation of a medicament for peripheral administration for use in the suppression of appetite or induction of satiety.

In a further preferred embodiment, the present invention relates to the use of GLP-1(1-36) or an analogue thereof or the C-terminal amide of any of these for the preparation of a medicament for peripheral administration for use in the suppression of appetite or induction of satiety.

In a further preferred embodiment, the present invention relates to the use of GLP-1(1-35) or an analogue thereof or the C-terminal amide of any of these for the preparation of a medicament for peripheral administration for use in the suppression of appetite or induction of satiety.

In a further preferred embodiment, the present invention relates to the use of GLP-1(1-34) or an analogue thereof or the C-terminal amide of any of these for the preparation of a medicament for peripheral administration for use in the suppression of appetite or induction of satiety.

In a further preferred embodiment, the present invention relates to the use of GLP-1(7-45) or an analogue thereof or the C-terminal amide of any of these for the preparation of a medicament for peripheral administration for use in the suppression of appetite or induction of satiety.

In a further preferred embodiment, the present invention relates to the use of GLP-1 (7-39) or an analogue thereof or the C-terminal amide of any of these for the preparation of a medicament for peripheral administration for use in the suppression of appetite or induction of satiety.

In a further preferred embodiment, the present invention relates to the use of GLP-1(7-38) or an analogue thereof or the C-terminal amide of any of these for the preparation of a medicament for peripheral administration for use in the suppression of appetite or induction of satiety.

In a further preferred embodiment, the present invention relates to the use of GLP-1 (7-37) or an analogue thereof or the C-terminal amide of any of these for the preparation of a medicament for peripheral administration for use in the suppression of appetite or induction of satiety.

In a further preferred embodiment, the present invention relates to the use of GLP-1(7-36) or an analogue thereof or the C-terminal amide of any of these for the preparation of a medicament for peripheral administration for use in the suppression of appetite or induction of satiety.

In a further preferred embodiment, the present invention relates to the use of GLP-1(7-35) or an analogue thereof or the C-terminal amide of any of these for the preparation of a medicament for peripheral administration for use in the suppression of appetite or induction of satiety.

In a further preferred embodiment, the present invention relates to the use of GLP-1 (7-34) or an analogue thereof or the C-terminal amide of any of these for the preparation of a medicament for peripheral administration for use in the suppression of appetite or induction of satiety.

The analogues derived from the GLP-1 peptides specifically mentioned above contain a maximum of five, preferably a maximum of three, more preferred a maximum of two changes, *i*.*e*. substitutions and/or deletions and/or extensions in the molecule.

In a further preferred embodiment, the present invention relates to a pharmaceutical composition for peripheral administration, comprising one of the above-mentioned GLP-1 peptides, for the prophylaxis or treatment of diseases or disorders associated with impaired appetite regulation or feeling of hunger.

In a further preferred embodiment, the present invention relates to a pharmaceutical composition for peripheral administration, comprising one of the above-mentioned GLP-1 peptides, for the prophylaxis or treatment of obesity.

In a further preferred embodiment, the present invention relates to a method of treating or preventing diseases or disorders associated with impaired appetite regulation or feeling of satiety, the method comprising administering to an individual in need of such treatment an amount of a GLP-1 peptide as mentioned above sufficient to suppress appetite or induce satiety in said individual.

In a further preferred embodiment, the present invention relates to a method of treating or preventing obesity the method comprising administering to an individual in need of such treatment a sufficient amount of a GLP-1 peptide as mentioned above.

### BRIEF DESCRIPTION OF THE DRAWING

The present invention is further illustrated with reference to the appended drawing wherein
Fig. 1 shows the gel filtration chromatogram obtained in the first purification step in Example 3;
Fig. 2 shows the HPLC chromatogram obtained in the second purification step in Example 3 and
Fig. 3 shows an enlarged section of the chromatogram shown in Fig.2.

### DETAILED DESCRIPTION OF THE INVENTION

The GLP-1 peptides used according to the invention can be produced by chemical synthesis or - more conveniently - by a method which comprises culturing a host cell containing a DNA sequence encoding the peptide and capable of expressing the peptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements.

Suitable media are available from commercial suppliers or may be prepared according to published recipes (*e.g.* in catalogues of the American Type Culture Collection). The polypeptide produced by the cells may then be recovered from the culture medium by conventional procedures including separating the host cells from the medium by centrifugation or filtration, precipitating the proteinaceous components of the supernatant or filtrate by means of a salt, *e.g.* ammonium sulphate, purification by a variety of chromatographic procedures, *e.g.* ion exchange chromatography, gel filtration chromatography, affinity chromatography, or the like, dependent on the type of polypeptide in question.

The DNA sequence encoding the parent polypeptide may suitably be of genomic or cDNA origin, for instance obtained by preparing a genomic or cDNA library and screening for DNA sequences coding for all or part of the polypeptide by hybridization using synthetic oligonucleotide probes in accordance with standard techniques (see, for example, Sambrook, J., Fritsch, E. F. and Maniatis, T., Molecular Cloning: A Laboratory Manual, Cold Spring Harbor Laboratory Press, New York, 1989). The DNA sequence encoding the polypeptide may also be prepared synthetically by established standard methods, *e.g.* the phosphoamidite method described by Beaucage and Caruthers, Tetrahedron Letters 22 (1981), 1859 - 1869, or the method described by Matthes et al., EMBO Journal 3 (1984), 801 - 805. The DNA sequence may also be prepared by polymerase chain reaction using specific primers, for instance as described in US 4,683,202 or Saiki et al., Science 239 (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, *i*.*e*. a vector which exists as an extrachromosomal entity, the replication of which is independent of chromosomal replication, *e.g.* a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the polypeptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the polypeptide of the invention in a variety of host cells are well known in the art, cf. for instance Sambrook *et al*., *supra*.

The DNA sequence encoding the polypeptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g.* a gene the product of which complements a defect in the host cell or one which confers resistance to a drug, *e.g.* ampicillin, kanamycin, tetracyclin, chloramphenicol, neomycin, hygromycin or methotrexate.

To direct a parent polypeptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the polypeptide in the correct reading frame. Secretory signal sequences are commonly positioned 5' to the DNA sequence encoding the polypeptide. The secretory signal sequence may be that normally associated with the polypeptide or may be from a gene encoding another secreted protein.

The procedures used to ligate the DNA sequences coding for the present polypeptide, the promoter and optionally the terminator and/or secretory signal sequence, respectively, and to insert them into suitable vectors containing the information necessary for replication, are well known to persons skilled in the art (cf., for instance, Sambrook *et al*., *supra*).

The host cell into which the DNA sequence or the recombinant vector is introduced may be any cell which is capable of producing the present polypeptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

Compounds which can be useful as GLP-1 peptides according to the present invention are described in International Patent Application No. WO 87/06941 (The General Hospital Corporation) which relates to a peptide fragment which comprises GLP-1(7-37) and functional derivatives thereof and to its use as an insulinotropic agent.

Further GLP-1 analogues are described in International Patent Application No. 90/11296 (The General Hospital Corporation) which relates to peptide fragments which comprise GLP-1(7-36) and functional derivatives thereof and have an insulinotropic activity which exceeds the insulinotropic activity of GLP-1(1-36) or GLP-1(1-37) and to their use as insulinotropic agents.

International Patent Application No. 91/11457 (Buckley et al.) discloses analogues of the active GLP-1 peptides 7-34, 7-35, 7-36, and 7-37 which can also be useful as GLP-1 peptides according to the present invention.

### Pharmaceutical compositions

Pharmaceutical compositions containing a GLP-1 peptide according to the present invention may be administered parenterally to patients in need of such a treatment. Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a powder or a liquid for the administration of the peptide in the form of a nasal or pulmonal spray. As a still further option, the peptide of the invention can also be administered transdermally, *e.g.* from a patch, optionally from a iontophoretic patch. Compositions suitable for buccal, rectal and vaginal administration may also be provided.

Pharmaceutical compositions containing a GLP-1 peptide of the present invention may be prepared by conventional techniques, *e.g.* as described in Gennaro, Alfonso R.(Editor) Remington: The Science and Practice of Pharmacy, Vol. 1-2, 19th Ed., Mack Publishing Company (1995).

Thus, the injectable compositions of the GLP-1 peptide of the invention can be prepared using the conventional techniques of the pharmaceutical industry which involves dissolving and mixing the ingredients as appropriate to give the desired end product.

Thus, according to one procedure, the GLP-1 peptide is dissolved in an amount of water which is somewhat less than the final volume of the composition to be prepared. An isotonic agent, a preservative and a buffer is added as required and the pH value of the solution is adjusted - if necessary - using an acid, *e.g.* hydrochloric acid, or a base, *e.g.* aqueous sodium hydroxide as needed. Finally, the volume of the solution is adjusted with water to give the desired concentration of the ingredients.

Examples of isotonic agents are sodium chloride, mannitol and glycerol.

Examples of preservatives are phenol, m-cresol, methyl p-hydroxybenzoate and benzyl alcohol.

Examples of suitable buffers are sodium acetate and sodium phosphate.

A composition for nasal administration of peptides may, for example, be prepared as described in European Patent No. 272097 (to Novo Nordisk A/S) or in WO 93/18785.

According to some embodiments of the present invention, the GLP-1 peptide is provided in the form of an injectable solution. In such embodiments, the solutions preferably contain not less than about 2 mg/ml, preferably not less than about 5 mg/ml, more preferred not less than about 10 mg/ml of the GLP-1 peptide and, preferably, not more than about 100 mg/ml of the GLP-1 peptide.

The GLP-1 peptides of this invention can be used in the treatment of various diseases. In particular, it is envisaged that the GLP-1 peptides will be useful for the preparation of a medicament for peripheral administration in the treatment of obesity. The particular GLP-1 peptide to be used and the optimal dose level for any patient will depend on the disease to be treated and on a variety of factors including the efficacy of the specific GLP-1 peptide employed, the age, body weight, physical activity, and diet of the patient, on a possible combination with other drugs, and on the severity of the case. It is recommended that the dosage of the GLP-1 peptide of this invention be determined for each individual patient by those skilled in the art. In general, however, the dosage will be in the range of from about 10 µg per kg body weight per day to about 5 mg per kg body weight per day. The total daily dose may be administered in the form of two or more subdivisions thereof. In one preferred embodiment, the GLP-1 peptide is administered in the form of a pharmaceutical composition which has a protracted profile of action. In another preferred embodiment, the GLP-1 peptide is administered over a prolonged period. Such administration can, for example, be effected by infusion, by iontophoresis or from a transdermal patch.

The present invention is further illustrated by the following examples which, however, are not to be construed as limiting the scope of protection. The features disclosed in the foregoing description and in the following examples may, both separately and in any combination thereof, be material for realizing the invention in diverse forms thereof.

### EXAMPLES

### Example 1

### The effect of GLP-1(7-36)amide on food intake in normal human subjects.

Healthy human volunteers (20 subjects) were in a double-blind crossover trial given an iv infusion of 50 pmol/(kg·h) of GLP-1(7-36)amide or saline on separate, but otherwise identical experimental days. The infusion was started at 9.00 and lasted until 15.00. At 9.45 the subjects received a fixed amount of breakfast and at 14.15 they had lunch of an identical composition but *ad libitum.* The subjects rated their feeling of satiety, hunger, fullness and appetite on a visual analogue scale every half hour. The results are given below:
- Satiety:: GLP-1 > Saline, p = 0.006
- Hunger:: GLP-1 < Saline, p = 0.007
- Fullness:: GLP-1 > Saline, p = 0.010
- Appetite:: GLP-1 < Saline, p = 0.007

The energy intake of each subjects was measured. The intakes were 3.9 MJ with GLP-1 and 4.3 MJ with Saline resulting in a p-value of 0.009.

### Example 2

### Test method for measuring appetite suppression in mice.

Mice were deprived of their normal food for two days and given free access to a 20 % sucrose solution on the first day of food deprivation. After the two day food deprivation period, the mice were injected intraperitoneally with 0.5ml of a solution containing the test substance. Immediately after injection, individual mice were placed in one of eight 15 cm square test boxes with a stainless steel grid floor and a glass drinking tube which projected into the box. The drinking tube was connected to a reservoir containing a 20 % sucrose solution, and the interior of the drinking tube contained an electrode enabling the detection of drinking contacts with the solution by measuring the flow of a weak (undetectable) electric current through mice by means of an electronic apparatus connected to the drinking tube electrode and the stainless steel grid floor. Consumption of the sucrose solution was measured over a 10 minute period by electronically recording the total amount of contact with the sucrose solution during the test session. The degree of appetite suppression produced by a given test substance was determined by statistical comparison of the mean duration of sucrose consumption by control (vehicle treated) mice with that of mice treated with a test substance. The degree of appetite suppression in a treated group of mice was expressed as percent difference between the test and control group's response duration means.

The test substance was GLP-1(7-36)amide dissolved in sterile saline. The test solution containing 85 micrograms of GLP-1(7-36)amide (equivalent to 3.4 mg/kg) suppressed sucrose consumption by 60% with a p-value of 0.002.

### Example 3.

### Suppression of food intake by GLP-1 peptides isolated from tumors in anorectic rats.

### Acid ethanol extraction of tumor tissue.

Anorectic tumors were produced in rats as previously described (Madsen, O.D. et al. (1993) Endocrinology 133 2022-2030). Fifty anorectic 12C3AN (MSL-G-AN) tumours (at -80°C) corresponding to 50.07 g of wet tissue were homogenised at 4°C with 700 ml of acid ethanol (96% ethanol/0.7M HCl, 3/1, vol/vol). The homogenisation was carried out for 5 min in a pre-cooled (4°C) 2 litre Waring Commercial Blender at maximum speed. After homogenisation the mixture was stirred at 4°C for 16 hours. The mixture was centrifuged at 9000 RPM at 4°C for 1 hour. The volume of the supernatant was reduced to 20% by vacuum evaporation. During this process, in which the main part of the ethanol is removed, some precipitate is formed. This precipitate was removed by centrifugation at 4°C for one hour at 20.000 RPM. The supernatant, which still contained some lipid-like material, was filtered and applied to a LiChroprep RP-18 (Merck) column (2.5 x 10cm) equilibrated with 0.1% TFA at a flow rate of 2 ml/min. The column was washed with 100 ml of 0.1% of TFA at a flow rate of 4 ml/min. Bound material was eluted with 400 ml of 0.1% TFA containing 70% (vol/vol) of acetonitrile. The acetonitrile was removed by vacuum evaporation and the resulting mixture was lyophilised. After lyophilisation, the material was dissolved in 50 ml of water and the pH was adjusted to 5.3 with 425 µl of 1N NaOH. Further titration of the mixture to pH 6.0 resulted in the formation of a precipitate. Upon back titration to pH 5.3 this precipitate was dissolved again. Therefore the pH was left at 5.3 and the mixture was lyophilised. The total yield of lyophilised material from 50 tumours was 359 mg of dry powder.

### First purification step: gel filtration on Sephadex G-75

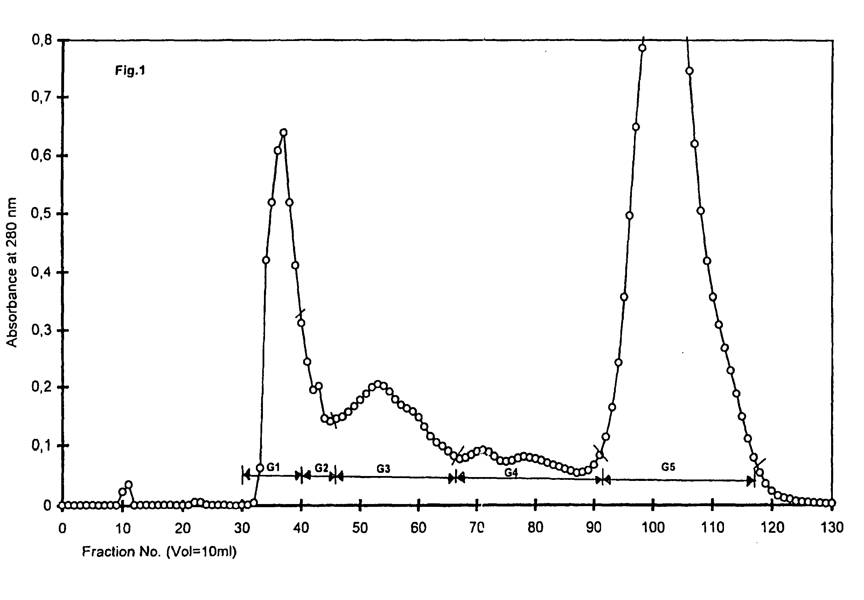

Lyophilised material (278 mg) from the acid ethanol extract corresponding to 38 individual tumors was redissolved in 20 ml of 1 M acetic acid and applied to Sephadex G75 column (5 x 50 cm). The column was equilibrated and eluted with 1 M acetic acid at a flow rate of 55 ml/h, and fractions corresponding to 10 ml were collected. The absorption at 280 nm was recorded for each fraction. The gel filtration chromatogram is shown in Fig. 1. Individual fractions were pooled in the following 5 main fractions: G1 (Fr. 30-39), G2 (Fr. 40-45), G3 (Fr. 46-66), G4 (Fr. 67-91) and G5 (Fr. 92-118) and subjected to bioassay after lyophilisation.

### Second purification step: Preparative HPLC of the G4 pool

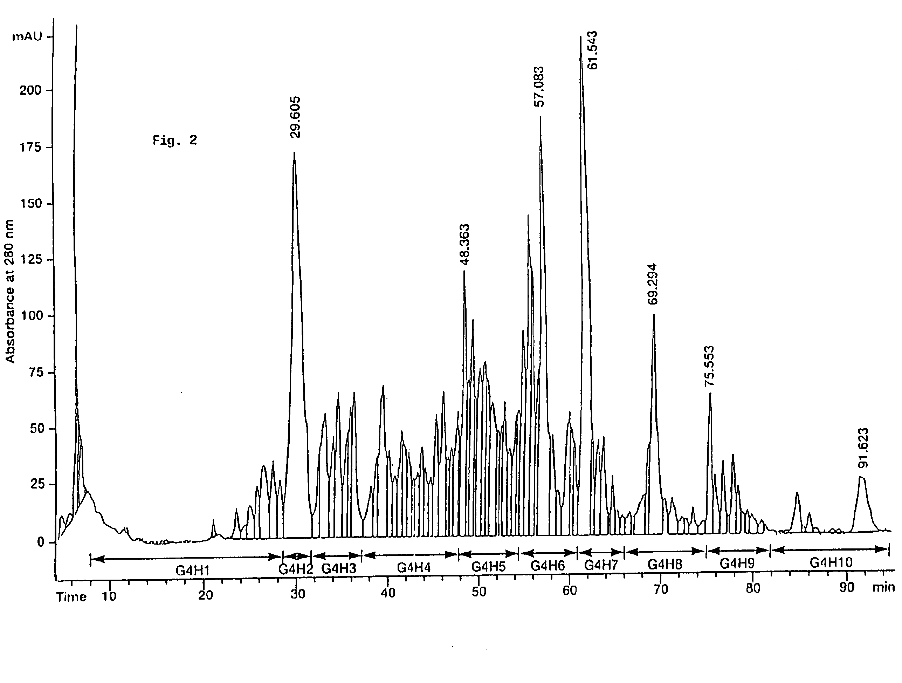

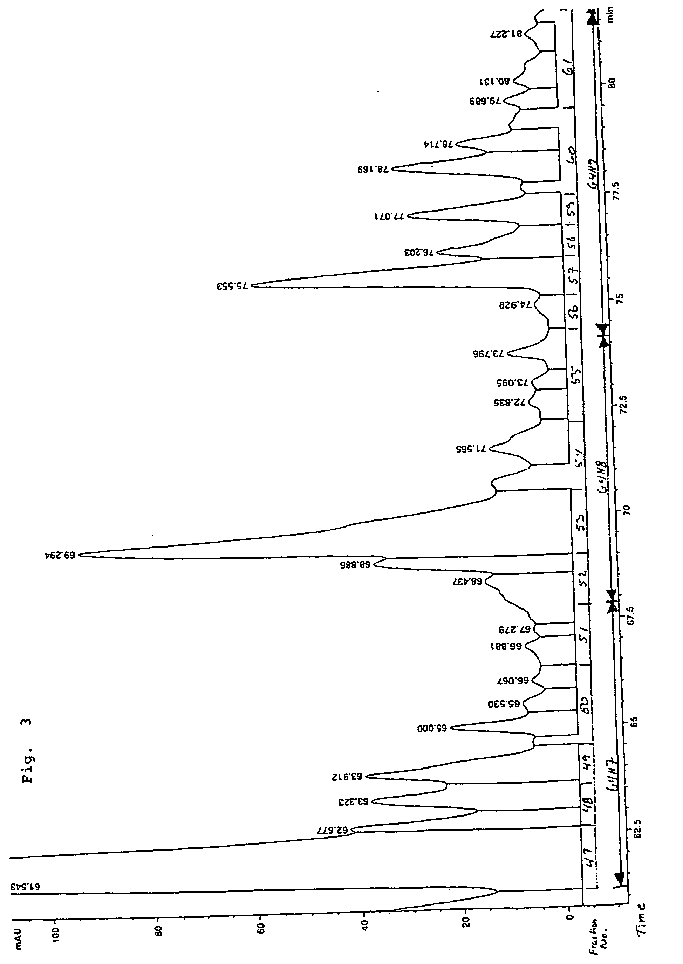

Some of the appetite suppression activity of the gel filtration pools showed the activity to be present in the G4 pool, and this pool was further fractionated by preparative HPLC. Lyophilised G4 material (corresponding to 80 tumors) was redissolved in 15 ml 0.1% TFA and pumped onto a Vydac 214TP1022 C4 column (2.2 x 25 cm) equilibrated in 0.1% TFA. The column was washed with 20 ml of 0.1% TFA, followed by 100 ml of MeCN/H₂O/TFA (10.0:89.9:0.1, v/v/v). The material was eluted at 25°C at a flow rate of 4 ml/min with a linear gradient formed from MeCN/H₂O/TFA (10:79.9:0.1, v/v/v) and MeCN/H₂O/TFA (65.0:34.9:0.1, v/v/v) over 110 min. UV absorption was monitored at 214 nm and 280 nm. The HPLC chromatogram (monitored at 280nm) is shown in Fig. 2 and in a larger magnification in Fig.3. Fractions corresponding to 61 pools were generated as indicated in Fig. 3. The volume was reduced to approx. 25% by vacuum evaporation and the fractions were lyophilised and tested in the bioassay.
The appetite suppression activity was found in fraction 53, 57 and 60 and the peptides of these fractions were analysed by amino acid sequence analysis and mass spectrometry analysis.

### Chemical characterisation of the peptides in fractions 53, 57 and 60

Amino acid sequence analysis was carried out by automated Edman degradation using an Applied Biosystems Model 477 gas-phase sequencer, essentially as described by the manufacturer. Mass spectrometry analysis was performed using an API III LC/MS/MS system (Sciex, Thornhill, Ont., Canada). The triple quadrupole instrument had a mass-to-charge (m/z) range of 2400 and was fitted with a pneumatically assisted electrospray (also referred to as ion-spray) interface (Bruins, A.P., Covey, T.R., & Henion, J.D. (1987) Anal. Chem. 59, 2642-2646 and Covey, T.R., Bonner, R.F., Shushan, B.I., & Henion, J.D. (1988) Rapid Commun. Mass Spectrom. 2, 249-256). Sample introduction was done by a syringe infusion pump (Sage Instruments, Cambridge, MA) through a fused capillary /75 mm i.d.) with a liquid flow rate set at 0.5-1 ml/min. The instrument m/z scale was calibrated with the singly-charged ammonium adduct ions of poly(propylene glycols) (PPGs) under unit resolution. The accuracy of mass measurements is generally better than 0.02%.

### Fraction 53:

By mass spectrometry it was found that the molecular weight of the peptide in this fraction was 3298. The amino acid sequence showed that the peptide was rat GLP-1(7-36)amide.

### Fraction 57:

By mass spectrometry it was found that the molecular weight of the peptide in this fraction was 3796. The amino acid sequence showed that the peptide was rat GPL-2 (1-33).

### Fraction 60:

The peptide in this fraction was found to be a mixture of rat GLP-1(1-36)amide and rat GLP-1(1-37), respectively.

### Test method for measuring appetite suppression in mice.

Mice were deprived of their normal food for two days and given free access to a 20% sucrose solution on the first day of food deprivation. After the two day food deprivation period, the mice were injected intraperitoneally with 0.5 ml of a solution containing the test substance. Thirty minutes after injection, individual mice were placed in one of eight 15 cm square test box with a stainless steel grid floor and a glass drinking tube which projected into the box. The drinking tube was connected to a reservoir containing a 20% sucrose solution, and the interior of the drinking tube contained an electrode enabling the detection of drinking contacts with the solution by measuring the flow of a weak (undetectable) electric current through mice by means of an electronic apparatus connected to the drinking tube electrode and the stainless steel grid floor. Consumption of the sucrose solution was measured over a 10 minute period by electronically recording the total amount of contact with the sucrose solution during the test session. The degree of appetite suppression produced by a given test substance was determined by statistical comparison of the duration of sucrose consumption by control (vehicle treated) mice with that of mice treated with a test substance. The degree of appetite suppression in a treated group of mice was expressed as percent of the control groups response.

### Test for appetite suppression in mice by fractions containing GLP-1(7-36)amide, GLP-1(1-36)amide and GLP-1(1-37).

Mice were tested for appetite suppression after treatment with a test substance. The test substance consisted of extracts of the anorectic glucagonoma tumor prepared by gel filtration (fraction G4) or by HPLC (fraction 53, 57 and 60) dissolved in phosphate buffered saline. The test solution containing lyophilised material from the Gel filtration fraction G4 corresponding to 3.3 tumors suppressed sucrose consumption by 72%. Of the 61 HPLC fractions of the G4 Gel filtration fraction (Fig. 2 and Fig.3), only the following 3 fractions gave a statistically significant suppression of appetite, when lyophilised material corresponding to 10 tumors was given:
Fraction 53, containing GLP-1(7-36)amide gave a suppression of food intake corresponding to 43%; fraction 57 containing GLP-2 gave a suppression of food intake corresponding to 41% ; fraction 60 containing a mixture of GLP-1(1-36)amide and GLP-1(1-37) gave a suppression of food intake corresponding to 41%.

### Example 4.

### Suppression of appetite in mice by systemically injected synthetic GLP-1(7-36)amide.

The test method was as described in Example 3 except that the mice were pre-exposed to formula milk (Complan® - a nutritionally complete infant milk substitute) for one day, then fasted one day. On the test day, consumption of Complan® was measured for each mouse during a 10 min period.

Mice were tested for appetite suppression by subcutaneous injection of GLP-1(7-36)amide dissolved in phosphate buffered saline. GLP-1(7-36)amide produced robust inhibitory effects on feeding 30 min after injection of as little as 1 mg/kg (45 % suppression, p < 0.001), and a maximum effect of 60% suppression (p < 0.001) was achieved with the highest dose tested, 20 mg/kg.

## Claims

1. Use of GLP-1 (7-38), or GLP-1 (7-39), or an analogue thereof for the preparation of a medicament for peripheral administration for use in the suppression of appetite or induction of satiety in a human subject, wherein the analogue differs from GLP-1 (7-38) or GLP-1 (7-39) by a maximum of five amino acid changes by substitution and/or addition.

2. Use as claimed in Claim 1 where the analogue differs from GLP-1 (7-38) or GLP-1 (7-39) by a maximum of three changes by substitution and/or addition.

3. Use as claimed in Claim 2 wherein the analogue differs from GLP-1 (7-38) or GLP-1 (7-39) by a maximum of two changes by substitution and/or addition.

4. Use of the C terminal amide of any of the compounds mentioned in any one of Claims 1 to 3 for the preparation of a medicament for peripheral administration for use in the suppression of appetite or induction of satiety in a human subject.

## Patentansprüche

1. Verwendung von GLP-1(7-38) oder GLP-1(7-39) oder eines Analogons davon zur Herstellung eines Medikaments zur peripheren Verabreichung zur Verwendung bei der Unterdrückung des Appetits oder Herbeiführung von Sättigung in einem menschlichen Patienten, wobei sich das Analogon von GLP-1(7-38) oder GLP-1(7-39) durch maximal fünf Aminosäureveränderungen durch Substitution und/oder Addition unterscheidet.

2. Verwendung nach Anspruch 1, wobei sich das Analogon von GLP-1(7-38) oder GLP-1(7-39) durch maximal drei Aminosäureveränderungen durch Substitution und/oder Addition unterscheidet.

3. Verwendung nach Anspruch 2, wobei sich das Analogon von GLP-1(7-38) oder GLP-1(7-39) durch maximal zwei Aminosäureveränderungen durch Substitution und/oder Addition unterscheidet.

4. Verwendung des C-terminalen Amids von beliebigen der in einem der Ansprüche 1 bis 3 erwähnten Verbindungen zur Herstellung eines Medikaments zur peripheren Verabreichung zur Verwendung bei der Unterdrückung des Appetits oder Herbeiführung von Sättigung in einem menschlichen Patienten.

## Revendications

1. Utilisation de GLP-1 (7-38) ou de GLP-1 (7-39) ou d'un de leurs analogues pour la préparation d'un médicament pour administration périphérique destiné à être utilisé dans la suppression de l'appétit ou l'induction de la satiété chez un sujet humain, dans laquelle l'analogue diffère de GLP-1 (7-38) ou de GLP-1 (7-39) au maximum de cinq changements d'acides aminés par substitution et/ou addition.

2. Utilisation selon la revendication 1, dans laquelle l'analogue diffère de GLP-1 (7-38) ou de GLP-1 (7-39) au maximum de trois changements d'acides aminés par substitution et/ou addition.

3. Utilisation selon la revendication 2, dans laquelle l'analogue diffère de GLP-1 (7-38) ou de GLP-1 (7-39) au maximum de deux changements d'acides aminés par substitution et/ou addition.

4. Utilisation de l'amide C-terminal de l'un quelconque des composés mentionnés dans l'une quelconque des revendications 1 à 3, pour la préparation d'un médicament pour administration périphérique destiné à être utilisé dans la suppression de l'appétit ou l'induction de la satiété chez un sujet humain.
